# EUROPÄISCHE PATENTSCHRIFT

(11) **EP 3 645 462 B1**
(45) Veröffentlichungstag und Bekanntmachung des Hinweises auf die Patenterteilung: **02.08.2023**
(21) Anmeldenummer: 18732354.8
(22) Anmeldetag: 22.06.2018
(51) Int. Cl.: C01B 32/80, C07C 263/10, B01J 7/02, B01J 19/00, B01J 19/24

(54) **ENERGIEEFFIZIENTES VERFAHREN ZUR BEREITSTELLUNG VON GEREINIGTEM PHOSGEN-DAMPF**
ENERGY-EFFICIENT PROCESS FOR PROVIDING PHOSGENE STEAM
PROCÉDÉ À HAUTE PERFORMANCE ÉNERGÉTIQUE DESTINÉ À PRODUIRE DE LA VAPEUR DE PHOSGÈNE

(30) Priorität: 29.06.2017 EP 17178652
(43) Veröffentlichungstag der Anmeldung: 06.05.2020
(73) Patentinhaber: Covestro Intellectual Property GmbH & Co. KG, 51373 Leverkusen (DE)
(72) Erfinder: MERKEL, Michael, 40223 Düsseldorf (DE); EHRIG, Martin, 51373 Leverkusen (DE); KOBYLKA, Manfred, 51399 Burscheid (DE); MORBACH, Jan, 50823 Köln (DE); CARRASCOSA MAS, Maria, 50674 Köln (DE); JENS, Christian, Morten, 51061 Köln (DE)
(74) Vertreter: Levpat
(86) Internationale Anmeldenummer: PCT/EP2018/066746
(87) Internationale Veröffentlichungsnummer: WO 2019/002121

(56) Entgegenhaltungen:
- EP-A1- 0 570 799
- EP-A1- 1 371 634
- WO-A1-2012/110597
- WO-A1-2012/130788
- DE-A1- 3 327 274
- DE-A1- 10 260 084
- US-A- 3 226 410

## Beschreibung

Die vorliegende Erfindung betrifft ein energieeffizientes Verfahren zur Bereitstellung von gereinigtem Phosgen-Dampf aus Chlor und Kohlenmonoxid. Weitere Gegenstände der Erfindung sind eine Vorrichtung zur Herstellung von gereinigtem Phosgen-Dampf sowie die Verwendung des gereinigten Phosgen-Dampfs für die chemische Synthese.

Die DE10260084A1 betrifft die Auftrennung eines Stoffgemisches aus Chlorwasserstoff und Phosgen betrifft die. Die WO 2012/110597 A1 betrifft die Verwendung von flüssigem Chlorwasserstoff als Kältemittel in Verfahren zur Chlorherstellung. Die US 3,226,410 betrifft ein kontinuierliches Verfahren zur Herstellung aromatischer Isocyanate. Die EP 0 570 799 A1 betrifft ein Verfahren zur Herstellung von aromatischen Diisocyanaten. Die EP 1 371 634 A1 betrifft ein Verfahren zur Reinigung von Mischungen von Toluoldiisocyanat.

Die kontinuierliche Herstellung von Phosgen in sogenannten Phosgenvereinigern ist bekannt und z.B. in "Ullmann's Encyclopedia of Industrial Chemistry", 7. Auflage 2012, Band 26, S. 625 ff (Kap. 3, Production) beschrieben. Dort wird auch eine Variante der Phosgenerzeugung beschrieben, bei der die Phosgenerzeugung in einem ersten Reaktionsschritt bei erhöhten Temperaturen von 200 bis 300 °C erfolgt. Aufgrund der ungünstigen Gleichgewichtslage bei dieser hohen Temperatur, schließt sich ein zweiter Reaktionsschritt bei niedrigerer Temperatur an. Die Reaktion erfolgt bei Normaldruck oder leichtem Überdruck und das gasförmige Phosgen wird entweder direkt verwendet oder zunächst kondensiert und/oder in einem Lösungsmittel absorbiert um Phosgenlösung zu erzeugen.

DE3327274A1 beschreibt ebenfalls ein zweistufiges Verfahren zur Erzeugung von Phosgen, wobei die Abwärme der ersten Verfahrensstufe bei hoher Temperatur (Heißphosgenerzeugung) zur Erzeugung von Dampf genutzt wird und die austretenden Reaktionsgase anschließend auf 50 - 120 °C abgekühlt werden, bevor in einer weiteren Reaktionsstufe bei 50 - 100 °C, der Umsatz vervollständigt wird, so dass der Chlorgehalt im Rohphosgen auf < 50 Vol.-ppm sinkt.

Auf diese Weise ist es möglich, sehr effizient Phosgen herzustellen, das nur noch eine geringe Menge an Chlor enthält. Da Kohlenmonoxid, im Folgenden auch CO genannt, üblicherweise im stöchiometrischen Überschuss zur Phosgenerzeugung eingesetzt wird, enthält der Produktstrom aus den Phosgenvereinigern jedoch teilweise noch erhebliche Mengen an nicht umgesetztem Kohlenmonoxid.

In EP 1 640 341 A2 oder auch in DE 102007057462 A1 wird beschrieben, dass der Überschuss an CO stofflich genutzt werden kann und so die CO-Emissionen verringert werden können. Dazu wird das aus den Phosgenvereinigern austretende Rohprodukt zunächst einer Teilkondensation unterzogen wobei Phosgen kondensiert und CO enthaltende Restgase abgetrennt werden. Diese Restgase werden unter Zuführung von weiterem Chlor in wenigstens einem Nachvereiniger zu weiterem Phosgen umgesetzt und anschließend wird auch das im Nachvereiniger hergestellt Phosgen in wenigstens einem Nachkondensator kondensiert und nicht kondensierbare Restgase werden abgetrennt.

US 4,231,959 beschreibt ein ähnliches Verfahren. Dabei wird jedoch das nicht kondensierbare Restgas aus der Kondensation nicht in einen Nachvereiniger umgesetzt, sondern in den eigentlichen Phosgenvereiniger rezykliert. Hierzu bedarf es einer Druckerhöhung für die z.B. ein mechanischer Kompressor oder eine Strahlpumpe zum Einsatz kommt, in der z.B. eines der Frischgase als Treibmedium eingesetzt wird.

Nachteilig an diesen Verfahren zur Phosgenherstellung ist, dass entweder gasförmiges Phosgen mit erheblichen Anteilen an inertem CO als Produkt anfällt (Ullmann) oder aber Phosgen als flüssiger Strom anfällt (EP`341, DE`462 und US`959), wobei für die Kondensation eine große Kühlleistung erforderlich ist und zwar aufgrund des niedrigen Siedepunkts von Phosgen auf einem technisch ungünstigen Temperaturniveau. US`959 nennt z.B. -20 °C bis -30 °C bei 1,5 kg/cm² bis 4,25 kg/cm² für eine vollständige Kondensation. Zudem wird in vielen heute üblichen technischen Anwendungen von Phosgen, hier sei beispielsweise die Gasphasen-Phosgenierung von Aminen zur Herstellung von Isocyanaten erwähnt, das Phosgen als Gas eingesetzt, so dass das flüssige Phosgen später wieder verdampft werden muss.

In WO 2012/130788 A1 ist ein Verfahren zur Herstellung von Isocyanaten offenbart, bei dem phosgenhaltiges Reaktionsgemisch aus der Gasphasensynthese von Kohlenmonoxid und Chlor zu Phosgen in zwei Teilströme aufgetrennt wird, von denen einer flüssig ist und < 1 Gew.-% Kohlenmonoxid enthält, und der andere gasförmig ist und > 10 Gew.-% Kohlenmonoxid enthält. Weiterhin wird beschrieben, dass der flüssige Strom mittels einer Pumpe komprimiert werden kann, so dass die direkte Kopplung der Druckniveaus in der Phosgenerzeugung und der Gasphasenphosgenierung aufgehoben wird. Weiterhin werden in dieser Schrift auch verschiedene Arten der Energieintegration erwähnt. So kann z.B. der erste, kohlenmonoxidarme Phosgenstrom als Kühlmittel für die Phosgensynthese verwendet werden, wobei er verdampft und gegebenenfalls auch überhitzt wird. Auch wenn ein sicherheitstechnischer Vorteil gegenüber einer Kühlung des Phosgenreaktors mit Wasser erwähnt wird, bestehen durchaus auch Vorbehalte gegen die beschriebene Vorgehensweise. Die Verwendung von Phosgen auf der Mantelseite eines Wärmetauschers (z.B. eines Phosgenvereinigers) birgt das Risiko einer Außenleckage, so dass von einer solchen Verwendung eher abzuraten ist und auch beim komprimieren flüssigen Phosgens mittels einer Pumpe besteht die Gefahr von Leckagen.

Weiterhin beschreibt die WO 2012/130788 A1 auch die Möglichkeit, den Schritt der Teilkondensation und den der Verdampfung wärmetechnisch zu koppeln, wenn die Verdampfung auf einem niedrigeren Druckniveau ausgeführt wird als die Kondensation. Es wird lediglich erwähnt, dass eine Druckdifferenz von mehr als 10 mbar bevorzugt sei ohne hierfür irgendwelche Vorteile zu nennen.

Für den zweiten, an Kohlenmonoxid reichen Strom ist erwähnt, dass er bevorzugt in die Phosgenerzeugung rezykliert wird. Auf diese Weise wird zwar CO stofflich genutzt, aber dazu muss es komprimiert werden und in dem dann geschlossenen Kreislauf können sich Komponenten, insbesondere Sauerstoff anreichern, so dass in der Regel ein Teilstrom ausgeschleust und entsorgt wird.

Aufgabe der vorliegenden Erfindung war es deshalb, auf möglichst effiziente und sichere Weise ein CO-armes Phosgengas zur weiteren Verwendung in der chemischen Synthese bereitzustellen, wobei auf weitere Pumpen und Verdichtungsschritte für das Phosgen verzichtet werden kann.

Diese Aufgabe konnte gelöst werden durch ein Verfahren zur Herstellung von gereinigtem Phosgen-Dampf, umfassend die Schritte
1) Bereitstellung eines aus der Umsetzung von Chlor mit Kohlenmonoxid erhältlichen Gasstroms umfassend Phosgen und Kohlenmonoxid,
2) Ein- oder mehrstufige Kondensation des Gasstroms und Abtrennung nicht kondensierbarer Restgase,
3) Ein- oder mehrstufige Verdampfung des in Schritt 2) erhaltenen flüssigen Phosgens und gegebenenfalls Überhitzung des erzeugten Phosgen-Dampfs,
wobei eine Energieintegration zwischen einer oder mehrerer der Kondensationsstufen von Schritt 2) und einer oder mehrerer der Verdampfungsstufen in Schritt 3) erfolgt und der Druck in der letzten Kondensationsstufe zwischen ≥ 0,2 und ≤ 6,0 bar höher liegt als in der ersten Verdampfungsstufe.

Vorliegend wird unter dem Begriff "Energieintegration" verstanden, dass die bei der Kondensation freiwerdende Wärme zumindest anteilig direkt und/oder indirekt zur Verdampfung genutzt wird.

Vorliegend lassen sich die in der Einheit "bar" angegebenen Drücke anhand der allgemein bekannten Umrechnungsfaktoren in die entsprechenden Werte der Einheit Pascal ("Pa") umrechnen, dabei gilt 1 bar ist gleich 100000 Pa.

In einer ersten bevorzugten Ausführungsform ist der Druck in der letzten Kondensationsstufe zwischen ≥ 0,3 und ≤ 4,5 bar, bevorzugt zwischen ≥ 0,5 und ≤ 3,0 bar, besonders bevorzugt zwischen ≥ 0,8 und ≤ 2,5 bar und ganz besonders bevorzugt zwischen ≥ 1,0 und ≤ 2,5 bar höher als in der ersten Verdampfungsstufe.

Die Bereitstellung des Gasstroms in Schritt 1) erfolgt bevorzugt aus einem Herstellungsverfahren unter Nutzung eines Teils der Reaktionswärme zur Erzeugung von Dampf (Heißphosgenerzeugung). Ein solches Verfahren ist beispielsweise in DE3327274A1 oder DE 102007057462 A1 ausführlich beschrieben. In einer weiteren bevorzugten Ausführungsform enthält der bereitgestellte Gasstrom neben Phosgen ≥ 1 Vol-% bis ≤ 20 Vol-%, bevorzugt ≥ 2 Vol-% bis ≤ 12 Vol-% und besonders bevorzugt ≥ 3 Vol-% bis ≤ 10 Vol-% CO. Der Gehalt an CO kann beispielsweise mittels IR Photometrie ermittelt werden. Dies liegt daran, dass durch einen CO Überschuss in der Phosgensynthese ein möglichst vollständiger Umsatz von Chlor erreicht werden soll. Chlor selbst ist dann meist nur in Spuren von < 50 Vol.-ppm enthalten. Der Druck in der Phosgenerzeugung wird bevorzugt so hoch gewählt, dass ein Druckgefälle von den Phosgenvereinigern über die Kondensationsstufe und die Verdampfungsstufe bis hin zur späteren Verwendung in der chemischen Synthese vorliegt.

In einer weiteren bevorzugten Ausführungsform werden die kondensierbaren Bestandteile des in Schritt 1) bereitgestellten Rohphosgens in Schritt 2) in einem ersten Kondensator bei einem Druck zwischen ≥ 2,0 bar(a) und ≤ 10,0 bar(a), bevorzugt zwischen ≥ 2,5 bar(a) und ≤ 6,0 bar(a) und ganz besonders bevorzugt zwischen ≥ 3,0 bar(a) und ≤ 5,0 bar(a) zumindest teilweise kondensiert. Der Fachmann erkennt, dass bei einem niedrigeren Druck in der Kondensation natürlich auch die erreichbare Druckdifferenz zwischen Kondensation und Verdampfung eingeschränkt ist, so dass sie in diesem Fall jeweils eher im unteren Bereich der oben genannten Vorzugsbereiche liegen wird.

Als Kondensator eignet sich für diesen Schritt ein Wärmetauscher, vorzugsweise ein Rohrbündelwärmetauscher. Bevorzugtes Material für den Wärmetauscher ist Edelstahl. In einer bevorzugten Ausführungsform befinden sich das Rohphosgen in den Rohren des Wärmetauschers und ein Kühlmedium auf der Mantelseite. Diese Ausführungsform minimiert das Risiko eines Phosgen-Austritts nach außen.

Vorzugsweise wird der gesamte phosgenhaltige Prozessstrom aus diesem Wärmetauscher in einen weiteren Kondensator geleitet, in dem eine weitere Kondensation erfolgt. Für diesen weiteren Kondensationsschritt eignen sich beispielsweise die bereits in der ersten Kondensationsstufe beschriebenen Wärmetauscher. Die Temperatur in der letzten Kondensationsstufe beträgt bevorzugt zwischen ≥ -60°C und ≤ 0°C, besonders bevorzugt zwischen ≥ -35 °C und ≤ -5°C und ganz besonders bevorzugt zwischen ≥ -20 °C und ≤ -10°C. Eine solche mehrstufige Prozessführung führt zu einem stabileren, weniger schwankungsanfälligen Verfahren. Darüber hinaus ist eine mindestens zweistufige Kondensation hilfreich, um den Prozess und die erfindungsgemäße Energieintegration anzufahren und für eine möglichst vollständige Kondensation des Phosgens zu sorgen. Bevorzugt erfolgt die Kondensation zweistufig. So lassen sich die genannten Vorteile mit geringem apparativem Aufwand realisieren.

In einer weiteren bevorzugten Ausführungsform des erfindungsgemäßen Verfahrens werden die nicht kondensierbaren Restgase aus Schritt 2), welche vorzugsweise CO als Hauptbestandteil enthalten, in mindestens einem Nachvereiniger mit Chlor zur weiteren Umsetzung zu Phosgen gebracht. Unter einem Nachvereiniger versteht man vorliegend also einen Nachreaktor zur weiteren Umsetzung von Kohlenmonoxid und Chlor zu Phosgen. Als Hauptbestandteil des Restgases ist CO vorliegend dann zu betrachten, wenn keine andere Verbindung einen höheren Stoffmengenanteil im Restgas einnimmt. Bevorzugt bestehen die nicht kondensierbaren Restgase zu ≥ 50 mol-% aus CO. Bevorzugt werden die nicht kondensierbaren Restgase dem am weitesten stromabwärts gelegenen Kondensator entnommen. Geeignete Nachvereiniger sind beispielsweise in DE 102007057462 A1 Absatz [0011] beschrieben. Dabei wird der CO-Gehalt des Abgases aus dem zweiten Kondensator kontinuierlich überwacht und die zugeführte Menge an Chlorgas für die Nachvereinigung entsprechend so geregelt, dass ein molarer CO-Überschuss gegenüber Chlor von ≥ 1,5 bis ≤ 20%, bevorzugt ≥ 2 bis ≤ 12% im Gasgemisch zum Nachvereiniger vorliegt. Die Überwachung des CO-Gehalts kann beispielsweise mittels IR-Photometrie erfolgen.

Das in den Kondensatoren in Schritt 2) anfallende flüssige Phosgen wird beispielsweise über eine Rohrleitung mit Druckhalteventil in die erste Verdampfungsstufe geleitet. Dabei ist es bevorzugt, die Flüssigkeitsmenge so gering wie möglich zu halten, um den Phosgen-Bestand in der Anlage nicht unnötig zu vergrößern. Außerdem ist es vorteilhaft, die dem Fachmann bekannten Maßnahmen zu ergreifen, um einen Durchschlag von gasförmigem Phosgen in den Verdampfer zu vermeiden. In einer besonders bevorzugten Ausführungsform des erfindungsgemäßen Verfahrens sind die Apparate räumlich so angeordnet, dass die Druckdifferenz zwischen den Kondensatoren und den Verdampfern ausreicht, um das flüssige Phosgen in die Verdampfungsstufe zu fördern.

Als Verdampfer in Schritt 3) eignen die aus dem Stand der Technik bekannten Wärmetauscher, bevorzugt Rohrbündelwärmetauscher, besonders bevorzugt solche aus Edelstahl. Die Verdampfung erfolgt bei einem Druck, der zwischen ≥ 0,3 und ≤ 5,0 bar, bevorzugt zwischen ≥ 0,5 und ≤ 3,0 bar, besonders bevorzugt zwischen ≥ 0,8 und ≤ 2,5 bar und ganz besonders bevorzugt zwischen ≥ 1,0 und ≤ 2,5 bar geringer ist als der Druck auf der Rohphosgenseite des ersten Kondensators. Durch diese erfindungsgemäße Druckdifferenz wird einerseits ein hinreichender Temperaturunterschied zwischen Kondensationstemperatur und Verdampfungstemperatur gewährleistet, so dass die Energieintegration auf effiziente Weise und mit technisch sinnvollen Wärmeüberträgerflächen erfolgen kann. Andererseits ist somit der Druck in der vorgelagerten Phosgenerzeugung nach oben hin begrenzt, was wiederum die in der Anlage gehandhabte Phosgenmenge limitiert, den Aufwand für die Kompression der Edukte Chlor und Kohlenmonoxid begrenzt, einen Flüssigkeitsanfall in den Phosgenvereinigern verhindert und eine zusätzliche Verdichtung des gasförmigen Rohphosgenstroms vor der Kondensation obsolet macht. Vielmehr ist im erfindungsgemäßen Verfahren der üblicherweise zur Verfügung stehende Druck der Edukte zur Phosgenerzeugung, Chlor und Kohlenmonoxid, ausreichend, um die gasförmigen und flüssigen Phosgenströme an die gewünschten Stellen bis hin zu den nachgelagerten Prozessen zu fördern ohne dass es zusätzlicher Kompressionsschritte bedarf.

In einer weiteren bevorzugten Ausführungsform liegt der Druck in der Verdampfungsstufe (Schritt 3)) ≥ 1,5 bar(a), bevorzugt ≥ 1,8 bar(a), besonders bevorzugt oberhalb von ≥ 2,0 bar(a) und ganz besonders bevorzugt oberhalb von ≥ 2,5 bar(a). Auf diese Weise kann für viele Anwendungen des Phosgen-Dampfs in der chemischen Synthese auf eine Kompression des Phosgens, sei es als Dampf oder als Flüssigkeit vor dem Verdampfen, verzichtet werden. In einer bevorzugten Ausführungsform der Erfindung erfolgt die Verdampfung des Phosgens auf der Rohrseite eines RohrbündelWärmetauschers. Das erfindungsgemäße Druckgefälle lässt sich beispielsweise leicht über Regel- oder Druckhalteventile in den jeweiligen Ableitungen der Gase sowie über ein Druckhalteventil zwischen Kondensation (Schritt 2)) und Verdampfung (Schritt 3)) einstellen.

Die erfindungsgemäße Energieintegration kann nun beispielsweise derart erfolgen, dass eine Kondensationsstufe und eine Verdampfungsstufe in einem einzigen Apparat, beispielsweise einem Rohrbündelwärmetauscher integriert sind. In diesem Fall ist es bevorzugt, die Kondensation bei höherem Druck in den Rohren erfolgen zu lassen während die Verdampfung bei geringerem Druck auf der Mantelseite erfolgt. Durch den direkten Energie-Übergang ist eine solche Verfahrensweise besonders effizient.

In einer bevorzugten Ausführungsform erfolgt die Energieintegration mittels eines Wärmeträgerkreislaufs zwischen mindestens einem Kondensator aus Schritt 2) und mindestens einem Verdampfer aus Schritt 3). Hierbei werden Kondensation und Verdampfung in zwei getrennten Apparaten, z.B. Rohrbündelwärmetauschern, jeweils auf der Rohrseite durchgeführt und die Energieintegration über ein Wärmeträgermedium bewerkstelligt, das zwischen den beiden Apparaten zirkuliert wird. Der Vorteil einer solchen Ausführungsform ist, dass sich das Risiko einer Phosgen-Leckage nach außen verringert, ohne dass die Effizienz gegenüber der im vorigen Absatz genannten Verfahrensweise signifikant geschmälert wird. Als Wärmeträgermedium kommen bevorzugt wasserfreie Substanzen zum Einsatz, die auch bei niedrigen Temperaturen bis ca. -20 °C eine niedrige Viskosität aufweisen und gut pumpfähig bleiben. Ein bevorzugtes Wärmeträgermedium ist Chlorbenzol. Das Wärmeträgermedium wird in einer bevorzugten Ausführungsform mittels einer Pumpe zwischen den Wärmetauschern für die Kondensation und für die Verdampfung zirkuliert. In einer alternativen Ausführungsform ist die Zuleitung des Wärmeträgermediums zum Kondensator darüber hinaus mit einer Möglichkeit zur Kühlung versehen und/oder die Zuleitung zum Verdampfer mit einer Möglichkeit zur Beheizung. Dies ist insbesondere bei einer einstufigen Kondensation oder Verdampfung vorteilhaft, um den Prozess und die erfindungsgemäße Energieintegration anzufahren und eine möglichst vollständige Kondensation/Verdampfung des Phosgens zu gewährleisten.

In Weiterbildung der Erfindung kann sich eine Überhitzungsstufe anschließen, in der der Phosgen-Dampf in einem Wärmetauscher überhitzt wird. Auch hier bietet sich eine Energieintegration an. Wird gemäß der bevorzugten Ausführungsform der Gasstrom für Schritt 1) aus einer Heißphosgenerzeugung bereitgestellt, so kann die oben beschriebene Abkühlung der den Reaktor verlassenden Reaktionsgase zur Vermeidung von Chloreisenbrand hinreichend Energie für die Überhitzung liefern. Bevorzugt erfolgt die Energieintegration wiederum über einen Kreislauf eines Wärmeträgermediums. Besonders bevorzugt handelt es sich hierbei um ein wasserfreies Wärmeträgermedium, ganz besonders bevorzugt um Chlorbenzol. Um die Komplexität des Verfahrens möglichst gering zu halten, ist es vorteilhaft, das gleiche Wärmeträgermedium als Energieträger für die in den beiden vorherigen Absätzen beschriebenen Energieintegrationen zu nutzen.

Je nach Qualitätsanforderung (CO-Gehalt) an den nach dem erfindungsgemäßen Verfahren hergestellten gereinigten Phosgen-Dampf, kann zwischen verschiedenen Ausführungsformen der Erfindung gewählt werden. Sind höhere Gehalte an CO zulässig, so kann je nach gewähltem CO Überschuss der aus der Nachvereinigung erhaltene Phosgenstrom ohne weiteres direkt mit dem Phosgendampf aus dem Verdampfer oder gegebenenfalls Überhitzer vereinigt werden. In der bevorzugten Ausführungsform mit Nachvereiniger für die Restgase, bewirkt ein geringerer CO-Überschuss im Nachvereiniger dabei einen weiter reduzierten CO-Gehalt des hergestellten Phosgen-Dampfs.

Bei erhöhten Qualitätsanforderungen erfolgt bevorzugt auch eine Kondensation (Nachkondensation) des aus dem Nachvereiniger austretenden Reaktionsgases wie in DE102007057462 Abschnitt [0030 und folgende] beschrieben. Das in dieser Nachkondensation erhaltene flüssige Phosgen wird bevorzugt dem in Schritt 2) erhaltenen kondensierten Phosgen hinzugefügt. Bevorzugt erfolgt diese Zugabe stromabwärts der Druckhalteeinrichtung, wo bereits der geringere Verdamfperdruck vorliegt, so dass auch hier keine Pumpe zur Förderung des Phosgens erforderlich ist.

Alternativ ist es mit dem erfindungsgemäßen Verfahren mit Nachvereiniger sogar möglich, Phosgendampf in verschiedenen Qualitätsstufen bereitzustellen. Dies kann vorteilhaft sein, wenn verschiedene nachgelagerte Prozesse mit unterschiedlichen Anforderungen, z.B. an den CO-Gehalt des Phosgens, bedient werden sollen. So ist es z.B. möglich, einen Teilstrom oder die Gesamtheit des durch Kondensation und Verdampfung gereinigten Phosgens für einen Prozess mit hohen Anforderungen zu entnehmen und den gegebenenfalls vorliegenden Reststrom zusammen mit dem weniger reinen Phosgenstrom aus dem Nachvereiniger für einen anderen, weniger anspruchsvollen Prozess zu verwenden.

Offenbart ist eine Vorrichtung zur Herstellung von gereinigtem Phosgen-Dampf aus der Umsetzung von Chlor mit Kohlenmonoxid, umfassend
- eine Kondensationseinrichtung zur teilweisen Kondensation eines Phosgen und Kohlenmonoxid umfassenden Gasstroms, die mindestens
   - eine erste Zuleitung für den Phosgen und Kohlenmonoxid umfassenden Gasstrom und
   - eine erste Ableitung für nicht kondensierte Gase aufweist,
- eine Verdampfungseinrichtung zur Verdampfung der in der Kondensationseinrichtung kondensierten Flüssigkeit, die mindestens
   - eine zweite Ableitung für einen gasförmigen Phosgenstrom aufweist,
- eine von der Kondensationseinrichtung abgehende und in die Verdampfungseinrichtung mündende Verbindungsleitung zur Förderung der in der Kondensationseinrichtung kondensierten Flüssigkeit und
- eine Einrichtung zur Übertragung von Wärme aus der Kondensationseinrichtung in die Verdampfungseinrichtung umfassend
   - mindestens eine Wärmeübertragungsfläche, die durch eine der Kondensationseinrichtung und der Verdampfungseinrichtung gemeinsame Wandung gebildet wird oder
   - mindestens einen Sekundärkreislauf der mit beiden Räumen jeweils mindestens eine gemeinsame Wandung aufweist und der zur Beinhaltung eines Wärmeträgermediums eingerichtet ist,
   dadurch gekennzeichnet dass die Kondensationseinrichtung so eingerichtet ist, dass ein Überdruck gegenüber der Verdampfungseinrichtung von ≥ 0,2 und ≤ 6,0 bar erreicht wird.

Ein weiterer Gegenstand der vorliegenden Erfindung ist eine ist eine Vorrichtung zur Herstellung von gereinigtem Phosgen-Dampf aus der Umsetzung von Chlor mit Kohlenmonoxid, umfassend
- eine Kondensationseinrichtung zur teilweisen Kondensation eines Phosgen und Kohlenmonoxid umfassenden Gasstroms, die mindestens
   - eine erste Zuleitung für den Phosgen und Kohlenmonoxid umfassenden Gasstrom und
   - eine erste Ableitung für nicht kondensierte Gase aufweist, der eine zweite Einrichtung zur Druckhaltung zugeordnet ist,
- eine Verdampfungseinrichtung zur Verdampfung der in der Kondensationseinrichtung kondensierten Flüssigkeit, die mindestens
   - eine zweite Ableitung für einen gasförmigen Phosgenstrom aufweist,
- eine von der Kondensationseinrichtung abgehende und in die Verdampfungseinrichtung mündende Verbindungsleitung zur Förderung der in der Kondensationseinrichtung kondensierten Flüssigkeit, wobei der Verbindungsleitung optional eine erste Einrichtung zur Druckhaltung zugeordnet ist, und
- eine Einrichtung zur Übertragung von Wärme aus der Kondensationseinrichtung in die Verdampfungseinrichtung umfassend
   - mindestens eine Wärmeübertragungsfläche, die durch eine der Kondensationseinrichtung und der Verdampfungseinrichtung gemeinsame Wandung gebildet wird oder
   - mindestens einen Sekundärkreislauf der mit beiden Räumen jeweils mindestens eine gemeinsame Wandung aufweist und der zur Beinhaltung eines Wärmeträgermediums eingerichtet ist,
   dadurch gekennzeichnet dass die Kondensationseinrichtung so eingerichtet ist, dass ein Überdruck gegenüber der Verdampfungseinrichtung von ≥ 0,2 und ≤ 6,0 bar erreicht wird.

Bevorzugt ist die Kondensationseinrichtung so eingerichtet, dass der Überdruck gegenüber der Verdampfungseinrichtung ≥ 0,3 und ≤ 4,5 bar, bevorzugt zwischen ≥ 0,5 und ≤ 3,0 bar, besonders bevorzugt zwischen ≥ 0,8 und ≤ 2,5 bar und ganz besonders bevorzugt zwischen ≥ 1,0 und ≤ 2,5 bar erreicht wird.

Mit der erfindungsgemäßen Vorrichtung kann auf energieeffiziente Weise gereinigter Phosgen-Dampf aus einem Gasstrom enthaltend Phosgen und Kohlenmonoxid hergestellt werden.

In einer ersten bevorzugten Ausführungsform ist der Verbindungsleitung der erfindungsgemäßen Reinigungsvorrichtung eine erste Einrichtung zur Druckhaltung zugeordnet. Diese Einrichtung dient gleichzeitig dazu, die Strömung von der Kondensationseinrichtung zur Verdampfungseinrichtung zu vergleichmäßigen und das Durchschlagen der nicht kondensierbaren Gase zu verhindern.

In der erfindungsgemäßen Reinigungsvorrichtung ist der ersten Ableitung für nicht kondensierte Gase eine zweite Einrichtung zur Druckhaltung zugeordnet.

In einer weiteren bevorzugten Ausführungsform der erfindungsgemäßen Reinigungsvorrichtung ist der zweiten Ableitung für einen gasförmigen Phosgenstrom eine dritte Einrichtung zur Druckhaltung zugeordnet.

Als Druckhalteeinrichtung sowohl für das erfindungsgemäße Verfahren als auch für die erfindungsgemäße Vorrichtung können zum Beispiel Regelventile, Tauchungen, Siphons, Lochblenden, Drosselventile, Überströmventile, Druckhalteventile und/oder eine geschickte räumliche Anordnung der Apparate zum Einsatz kommen. Bevorzugt kommen als Druckhalteeinrichtungen Regelventile zum Einsatz, wobei die Regelkreise bevorzugt Drucksensoren enthalten. Dies bietet den Vorteil, dass mit der erfindungsgemäßen Vorrichtung eine genaue Regelung des Drucks in der Kondensations- und Verdampfungseinrichtung möglich ist. Außerdem bietet die Verwendung von Regelventilen den Vorteil, dass im Gegensatz zu Tauchungen oder Siphons kein oder nur ein geringer Flüssigkeitsstand benötigt wird, so dass die in der Vorrichtung gehandhabte Phosgenmenge nicht unnötig erhöht wird.

In einer weiteren bevorzugten Ausführungsform handelt es sich bei der Kondensationseinrichtung und der Verdampfungseinrichtung jeweils um die Rohrseite eines oder mehrerer Rohrbündelwärmetauscher.

Hierbei ist es besonders bevorzugt, dass die Einrichtung zur Übertragung von Wärme eine oder mehrere Leitungen und mindestens eine Zwangsförderungseinrichtung umfasst, wobei diese derart angeordnet sind, dass sich unter Einbeziehung der Mantelseiten der Rohrbündelwärmetauscher ein Kreislauf ergibt, in dem ein Wärmeträgermedium zirkuliert werden kann.

Phosgen ist beispielsweise für die Isocyanatherstellung aus Aminen wichtig, somit ist die Verwendung des gereinigten Phosgen-Dampfs aus dem erfindungsgemäßen Verfahren oder aus der erfindungsgemäßen Vorrichtung zur Phosgenierung von Aminen in der Gasphase offenbart. Die Gasphasenphosgenierung von Aminen ist beispielsweise aus der EP 0 289 840 A1 bekannt.

Die vorliegende Erfindung wird mit Bezug auf die nachfolgenden Figuren und Beispiele näher erläutert, ohne jedoch darauf beschränkt zu sein. Es ist dem Fachmann möglich, z.B. durch Erhöhung der Druckdifferenz zwischen Kondensation und Verdampfung oder aber durch eine höhere Wärmeübertragungsfläche die Energieeinsparung durch die Energieintegration zu maximieren.

**FIG. 1** beschreibt eine Ausführungsform der in WO2012130788A1 beschriebenen Phosgenreinigung. Aus dem Phosgenvereiniger (101) tritt ein gasförmiger Rohphosgenstrom (102) aus, der neben Phosgen noch überschüssiges Kohlenmonoxid enthält. Das Phosgen aus diesem Strom wird in zwei aufeinanderfolgenden Wärmetauschern (103, 104) teilweise kondensiert. Der gasförmige, CO-reiche Strom (105) wird in einem Kompressor (106) verdichtet und zusammen mit Chlor (107) und frischem CO (108) zurück in den Phosgenerzeuger (101) geleitet. Der flüssige Phosgenstrom (109) dient als Kühlmittel für den Wärmetauscher (103) in dem das Phosgen zuvor kondensiert wurde. Der (teilweise) verdampfte Phosgenstrom (110) wird in einem weiteren Wärmetauscher (111) überhitzt und dann als Stom (112) einer Gasphasenphosgenierung von Aminen zugeführt.

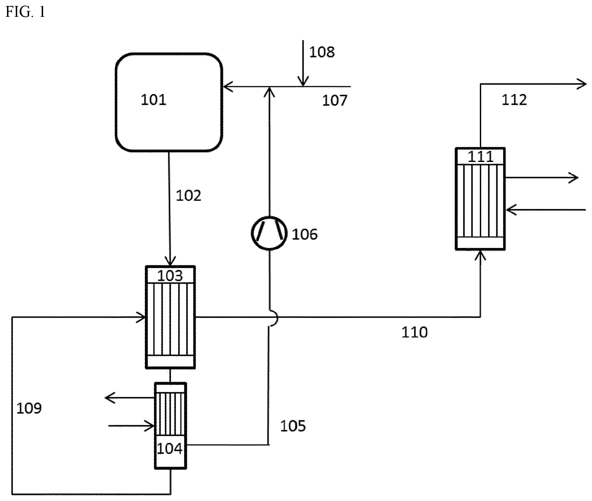

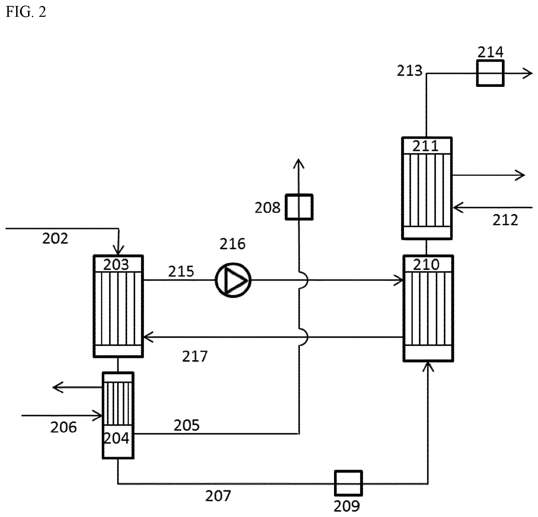

**FIG. 2** beschreibt eine bevorzugte Ausführungsform des erfindungsgemäßen Verfahrens. Der Rohphosgenstrom aus der Phosgenierung (202) wird in zwei aufeinanderfolgenden Wärmetauschern (203, 204) teilweise kondensiert, wobei der Wärmetauscher (204) mittels -13 °C kaltem Monochlorbenzol (206) gekühlt wird. Der nicht kondensierbare, CO-reiche Gasstrom (205) wird über ein Druckhalteventil (208) abgeführt und z.B. einer Abgasbehandlung zugeführt. Der flüssige Phosgenstrom (207) wird über eine Druckhalteeinrichtung (209) entspannt und dann in zwei aufeinanderfolgenden Wärmetauschern (210, 211) verdampft und überhitzt. Der Wärmetauscher (211) wird dabei mit heißem Monochlorbenzol (212) beheizt. Der überhitzte Phosgenstrom (213) wird über ein Regelventil (214), das auch der Druckregelung für den Verdampfungsschritt dient, seiner nachfolgenden Verwendung zugeführt. Die Wärmetauscher (203) und (204) sind mantelseitig Teil eines gemeinsamen Wärmeträgerkreislaufs. Im Wärmetauscher (203) durch die Kondensation aufgewärmtes Wärmeträgermedium (215) wird mittels einer Pumpe (216) zum Wärmetauscher (210) gefördert. Dort gibt es Wärme an das verdampfende Phosgen ab und kühlt sich dabei ab. Das abgekühlte Wärmeträgermedium (217) wird dann zurück zum Wärmetauscher (203) geleitet. Alternativ ist es natürlich ebenso denkbar, die Pumpe (216) in den kalten Wärmeträgerstrom (217) statt in den warmen Wärmeträgerstrom (215) zu integrieren.

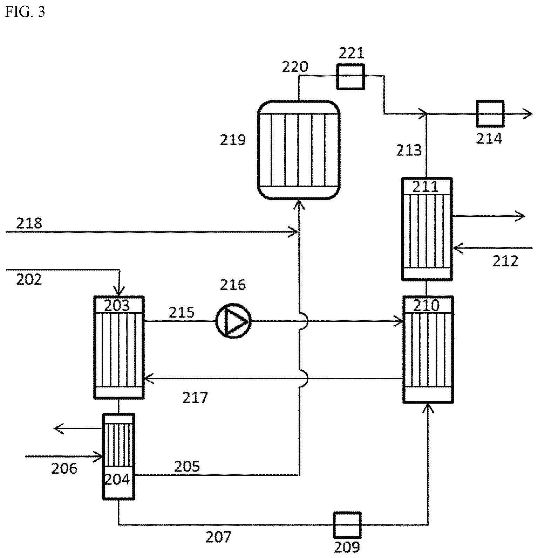

**FIG. 3** beschreibt eine weitere bevorzugte Ausführungsform des erfindungsgemäßen Verfahrens unter Verwendung eines Phosgennachvereinigers. In dieser Ausführungsform wird der nicht kondensierbare CO-reiche Gasstrom (205) mit Chlor (218) versetzt und in einem Nachvereiniger (219) zu weiterem Phosgen (220) umgesetzt, welches dann über ein Regelventil (221) dem überhitzten Phosgenstrom (213) zugeführt wird.

Auch beschreiben FIG. 2 und FIG. 3 bevorzugte Ausgestaltungen der erfindungsgemäßen Vorrichtung zur Reinigung von Phosgen. Daher sind die in FIG. 2 und FIG. 3 genannten Einrichtungen, Leitungen und Apparate inklusive deren Bezugszeichen ebenfalls Teil dieser bevorzugten Ausgestaltungen der erfindungsgemäßen Vorrichtung.

### Beispiele

Bei den Beispielen handelt es sich um Modellberechnungen die mittels Aspen Plus^{®} V8.8 erstellt wurden. Dabei wurde auf die in der Software hinterlegten Stoffdaten für CO und Phosgen zurückgegriffen (Stoffmodell Wilson/ideales Gas unter Verwendung der in Aspen Plus^{®} verfügbaren Datenbank AVP88-PURE24).

Die in den Beispielen genannten Kühlkurven für Kondensationsvorgänge und Heizkurven für die Verdampfungsvorgänge sind in den Figuren 4, 5 und 6 abgebildet. Es zeigen:
**FIG. 4** die Kühl- und Heizkurven aus den Beispielen 2 bis 4,
**FIG. 5** die Kühl- und Heizkurven aus den Beispielen 5 und 6 und
**FIG. 6** die Kühl- und Heizkurven aus den Beispielen 7 bis 9.

Dabei ist auf der x-Achse jeweils die Kühlleistung (Kondensation) bzw. die Heizleistung (Verdampfung) aufgetragen, wobei zur besseren Übersichtlichkeit beide Leistungsarten mit dem gleichen Vorzeichen versehen wurden. Auf der y-Achse ist dagegen jeweils die Temperatur in °C aufgetragen.

### Beispiel 1 (Taupunkte gasförmiger Gemische aus Kohlenmonoxid und Phosgen)

In Modellrechnungen wurden Gasgemische bestehend aus 15 mol-% CO und 85 mol-% Phosgen beziehungsweise 8 mol-% CO und 92 mol-% Phosgen von 60 °C auf -13 °C abgekühlt. Der Druck wurde variiert und der jeweilige Taupunkt dokumentiert.

| Druck [bar(a)] | Taupunkt 15% CO [°C] | Taupunkt 8% CO [°C] |
|---|---|---|
| 2 | 21,9 | 24,2 |
| 2,5 | 28,5 | 31,0 |
| 3 | 34,2 | 36,8 |
| 3,5 | 39,2 | 41,9 |
| 4 | 43,7 | 46,4 |
| 5 | 51,5 | 54,4 |
| 6 | 58,2 | 61,2 |
| 8 | 69,4 | 72,6 |
| 10 | 78,7 | 82,0 |

### Beispiel 2: (Kondensation bei 3,0 bar(a); 15% CO)

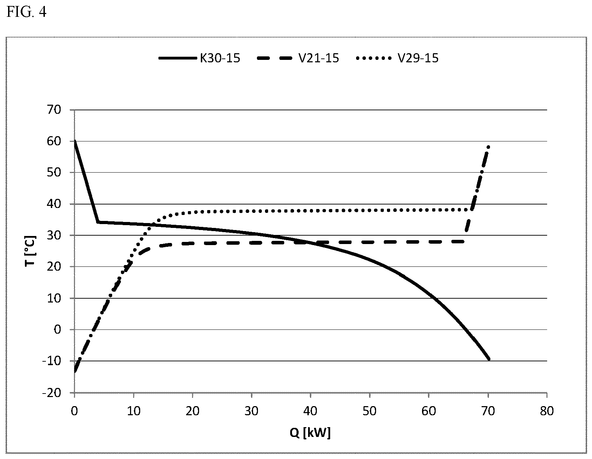

Ein 900 kg/h Strom der oben genannten Zusammensetzung mit 15 mol-% CO wurde in einer Flash-Berechnung bei 3,0 bar(a) von 60 °C auf -13 °C abgekühlt und in einen gasförmigen CO-reichen Strom und einen flüssigen phosgenreichen Strom aufgetrennt. Wie in Beispiel 1 gezeigt, setzte die Kondensation bei einem Taupunkt von 34,2 °C ein. Für die Abkühlung von 60 °C auf -13 °C war eine Kühlleistung von 71,5 kW erforderlich. Nur ein kleiner Teil dieser Energie wird der Überhitzung des Gasstroms entnommen. Der weitaus größere Teil stammt aus der Kondensation des Phosgens und fällt erst bei Temperaturen unterhalb des Taupunkts an (siehe Kühlkurve K30-15 in Fig. 4). Der flüssige Austrittstrom von 834 kg/h enthielt noch ca. 0,4% CO.

### Beispiel 3 (Vergleichsbeispiel: Verdampfung des flüssigen Stroms aus Beispiel 2 bei einem Druck von 2,9 bar(a), also 100 mbar unterhalb des Drucks der Kondensationsstufe)

Der bei 3,0 bar(a) und -13 °C kondensierte Phosgenstrom von 834 kg/h aus Beispiel 2 wurde einer erneuten Verdampfung und Überhitzung auf 60 °C unterzogen, wofür 70,3 kW Heizleistung benötigt wurden. Die Verdampfung erfolgte dabei bei einem Druck von 2,9 bar(a). Die zugehörige Heizkurve (V29-15 in Fig. 4)zeigt, dass der wesentliche Teil der Verdamfpungsenergie bei ca. 38 °C benötigt wird und damit oberhalb des Taupunkts (34,2 °C) des Gasgemisches aus Kohlenmonoxid und Phosgen unter diesen Bedingungen. Vervollständigt wird die Verdampfung bei ca. 38,2 °C (67,2 kW). Aus der Gegenüberstellung der Kühlkurve des Phosgen/CO-Gemisches bei 3,0 bar(a) (K30-15 in Fig. 4) und der Heizkurve des gereinigten Phosgens bei 2,9 bar(a) (V29-15 in Fig. 4) wird deutlich, dass eine Energieintegration bei der gewählten Druckdifferenz nicht sinnvoll ist, da bestenfalls Bruchteile der nötigen Energie für die Kondensation/Verdampfung auf die Wärmekopplung entfallen können.

### Beispiel 4 (erfindungsgemäßes Beispiel: Verdampfung des flüssigen Stroms aus Beispiel 2 bei einem Druck von 2,1 bar(a), also 0,9 bar unterhalb des Drucks der Kondensationsstufe)

Der bei 3,0 bar(a) und -13 °C kondensierte Phosgenstrom von 834 kg/h aus Beispiel 2 wurde einer erneuten Verdampfung und Überhitzung auf 60 °C unterzogen, wofür 70,3 kW Heizleistung benötigt wurden. Die Verdampfung erfolgte dabei bei einem Druck von 2,1 bar(a). Die zugehörige Heizkurve (V21-15 in Fig. 4) zeigt, dass der wesentliche Teil der Verdamfpungsenergie bei ca. 28 °C benötigt wird und damit unterhalb des Taupunkts (34,2 °C) des Gasgemisches aus Kohlenmonoxid und Phosgen unter diesen Bedingungen. Vervollständigt wird die Verdampfung bei ca. 28,1 °C (65,4 kW). Es wird deutlich, dass eine Energieintegration bei der gewählten Druckdifferenz von 0,9 bar sinnvoll ist, da ein signifikanter Teil der benötigten Kühl- und Heizenergie jeweils über die Kondensations- bzw. Verdampfungswärme im Rahmen der Energieintegration zur Verfügung gestellt werden kann.

### Beispiel 5: (Kondensation bei 4,0 bar(a); 15% CO)

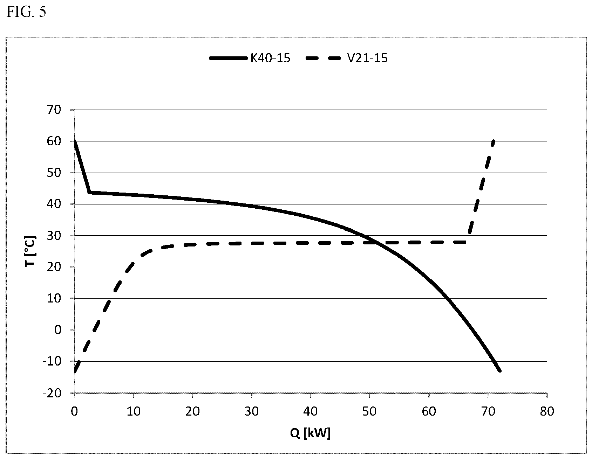

Ein 900 kg/h Strom der oben genannten Zusammensetzung mit 15 mol-% CO wurde in einer Flash-Berechnung bei 3,0 bar(a) von 60 °C auf -13 °C abgekühlt und in einen gasförmigen CO-reichen Strom und einen flüssigen phosgenreichen Strom aufgetrennt. Wie in Beispiel 1 gezeigt, setzte die Kondensation bei einem Taupunkt von 43,7 °C ein. Für die Abkühlung von 60 °C auf -13 °C war eine Kühlleistung von 72,0 kW erforderlich. Nur ein kleiner Teil dieser Energie wird der Überhitzung des Gasstroms entnommen. Der weitaus größere Teil stammt aus der Kondensation des Phosgens und fällt erst bei Temperaturen unterhalb des Taupunkts an (siehe Kühlkurve K40-15 in Fig. 5). Der flüssige Austrittstrom von 841 kg/h enthielt noch ca. 0,4% CO.

### Beispiel 6 (erfindungsgemäßes Beispiel: Verdampfung des flüssigen Stroms aus Beispiel 5 bei einem Druck von 2,1 bar(a), also 1,9 bar unterhalb des Drucks der Kondensationsstufe)

Der bei 4,0 bar(a) und -13 °C kondensierte Phosgenstrom von 841 kg/h aus Beispiel 3 wurde einer erneuten Verdampfung und Überhitzung auf 60 °C unterzogen, wofür 70,9 kW Heizleistung benötigt wurden. Die Verdampfung erfolgte dabei bei einem Druck von 2,1 bar(a). Die zugehörige Heizkurve (V21-15 in Fig. 5)zeigt, dass der wesentliche Teil der Verdamfpungsenergie bei ca. 28 °C benötigt wird und damit unterhalb des Taupunkts (34,2 °C) des Gasgemisches aus Kohlenmonoxid und Phosgen unter diesen Bedingungen. Vervollständigt wird die Verdampfung bei ca. 28,0 °C (66,4 kW). Es wird deutlich, dass eine Energieintegration bei der gewählten Druckdifferenz von 1,9 bar sinnvoll ist, da ein signifikanter Teil der benötigten Kühl- und Heizenergie jeweils über die Kondensations- bzw. Verdampfungswärme im Rahmen der Energieintegration zur Verfügung gestellt werden kann.

### Beispiel 7 (Kondensation bei 3,0 bar(a); 8% CO)

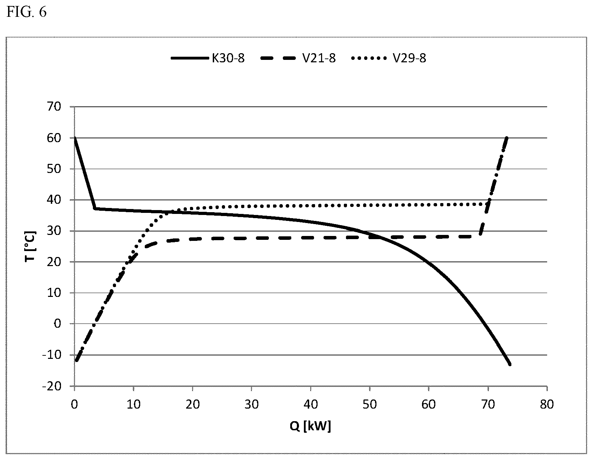

Ein 900 kg/h Strom der oben genannten Zusammensetzung mit 8 mol-% CO wurde in einer Flash-Berechnung bei 3,0 bar(a) von 60 °C auf -13 °C abgekühlt und in einen gasförmigen CO-reichen Strom und einen flüssigen phosgenreichen Strom aufgetrennt. Wie in Beispiel 1 gezeigt, setzte die Kondensation bei einem Taupunkt von 36,8 °C ein. Für die Abkühlung von 60 °C auf -13 °C war eine Kühlleistung von 73,7 kW erforderlich. Nur ein kleiner Teil dieser Energie wird der Überhitzung des Gasstroms entnommen. Der weitaus größere Teil stammt aus der Kondensation des Phosgens und fällt erst bei Temperaturen unterhalb des Taupunkts an (siehe Kühlkurve K30-8 in Fig. 6). Der flüssige Austrittstrom von 867 kg/h enthielt noch ca. 0,4% CO.

### Beispiel 8 (Vergleichsbeispiel: Verdampfung des flüssigen Stroms aus Beispiel 7 bei einem Druck von 2,9 bar(a), also 100 mbar unterhalb des Drucks der Kondensationsstufe)

Der bei 3,0 bar(a) und -13 °C kondensierte Phosgenstrom von 867 kg/h aus Beispiel 7 wurde einer erneuten Verdampfung und Überhitzung auf 60 °C unterzogen, wofür 73,1 kW Heizleistung benötigt wurden. Die Verdampfung erfolgte dabei bei einem Druck von 2,9 bar(a). Die zugehörige Heizkurve (V29-8 in Fig. 6) zeigt, dass der wesentliche Teil der Verdamfpungsenergie bei ca. 38 °C benötigt wird und damit oberhalb des Taupunkts (36,8 °C) des Gasgemisches aus Kohlenmonoxid und Phosgen unter diesen Bedingungen. Vervollständigt wird die Verdampfung bei ca. 38,2 °C (70,0 kW). Aus der Gegenüberstellung der Kühlkurve des Phosgen/CO-Gemisches bei 3,0 bar(a) (K30-8 in Fig. 6) und der Heizkurve des gereinigten Phosgens bei 2,9 bar(a) (V29-8 in Fig. 6) wird deutlich, dass eine Energieintegration bei der gewählten Druckdifferenz nicht sinnvoll ist, da bestenfalls Bruchteile der nötigen Energie für die Kondensation/Verdampfung auf die Wärmekopplung entfallen können.

### Beispiel 9 (erfindungsgemäßes Beispiel: Verdampfung des flüssigen Stroms aus Beispiel 7 bei einem Druck von 2,1 bar(a), also 0,9 bar unterhalb des Drucks der Kondensationsstufe)

Der bei 3,0 bar(a) und -13 °C kondensierte Phosgenstrom von 867 kg/h aus Beispiel 7 wurde einer erneuten Verdampfung und Überhitzung auf 60 °C unterzogen, wofür 73,1 kW Heizleistung benötigt wurden. Die Verdampfung erfolgte dabei bei einem Druck von 2,1 bar(a). Die zugehörige Heizkurve (V21-8 in Fig. 6) zeigt, dass der wesentliche Teil der Verdamfpungsenergie bei ca. 28 °C benötigt wird und damit unterhalb des Taupunkts (36,8 °C) des Gasgemisches aus Kohlenmonoxid und Phosgen. Vervollständigt wird die Verdampfung bei ca. 28,0 °C (68,5 kW). Es wird deutlich, dass eine Energieintegration bei der gewählten Druckdifferenz von 0,9 bar sinnvoll ist, da ein signifikanter Teil der benötigten Kühl- und Heizenergie jeweils über die Kondensations- bzw. Verdampfungswärme im Rahmen der Energieintegration zur Verfügung gestellt werden kann.

### Beispiel 10 (Vergleichsbeispiel: Kondensation bei 9,0 bar(a), Verdampfung bei 2,0 bar(a))

Diesem Beispiel liegt wiederum ein Gasstrom der Zusammensetzung 8 mol-% CO und 92 mol-% Phosgen zugrunde. Die Verdampfung soll nun bei 2 bar(a) erfolgen. Der Druckunterschied von 7 bar führt zu einem großen Temperaturunterschied zwischen Kondensations- und Verdampfungsstufe und ermöglicht somit theoretisch eine effiziente Energieintegration. Allerdings ist diese Verfahrensweise mit erheblichen Nachteilen verbunden. Dem in Beispiel 1 beschriebenen Zusammenhang zwischen Druck und Taupunkt kann entnommen werden, dass ein Druck von 9 bar(a) in der Kondensationsstufe mit einem Taupunkt von ca. 78 °C einhergeht. Bei geringeren CO-Überschüssen steigt der Taupunkt sogar noch weiter an. Da eine Kondensation in den stromaufwärts gelegenen Phosgenvereinigern unbedingt zu vermeiden ist, müssen diese bei erhöhten Temperaturen betrieben werden, die außerhalb der bekannten Vorzugsbereiche liegen (vgl. DE`462, Abschnitt 0025). Die Folge ist ein unerwünscht erhöhter Chlorgehalt im Phosgen. Alternativ könnte der Phosgenstrom zwischen der Phosgenerzeugung und der Kondensation verdichtet werden, was jedoch Energie und Apparaten für die Kompression bedarf und das Risiko einer Leckage erhöht. Zusätzlich müssten Maßnahmen gegen eine unkontrollierte Kondensation während der Kompression getroffen werden. Ein so großer Druckunterschied zwischen Kondensations- und Verdampfungsstufe ist also nicht erstrebenswert.

## Patentansprüche

1. Verfahren zur Herstellung von gereinigtem Phosgen-Dampf, umfassend die Schritte
1) Bereitstellung eines aus der Umsetzung von Chlor mit Kohlenmonoxid erhältlichen Gasstroms umfassend Phosgen und Kohlenmonoxid,
2) Ein- oder mehrstufige Kondensation des Gasstroms und Abtrennung nicht kondensierbarer Restgase,
3) Ein- oder mehrstufige Verdampfung des in Schritt 2) erhaltenen flüssigen Phosgens und gegebenenfalls Überhitzung des erzeugten Phosgen-Dampfs,
wobei eine Energieintegration zwischen einer oder mehrerer der Kondensationsstufen von Schritt 2) und einer oder mehrerer der Verdampfungsstufen in Schritt 3) erfolgt und der Druck in der letzten Kondensationsstufe zwischen ≥ 0,2 und ≤ 6,0 bar höher liegt als in der ersten Verdampfungsstufe.

2. Verfahren gemäß Anspruch 1, **dadurch gekennzeichnet, dass** der Druck in der letzten Kondensationsstufe zwischen ≥ 0,3 und ≤ 4,5 bar, bevorzugt zwischen ≥ 0,5 und ≤ 3,0 bar, besonders bevorzugt zwischen ≥ 0,8 und ≤ 2,5 bar und ganz besonders bevorzugt zwischen ≥ 1,0 und ≤ 2,5 bar höher liegt als in der ersten Verdampfungsstufe.

3. Verfahren gemäß einem der Ansprüche 1 oder 2, **dadurch gekennzeichnet, dass** der in Schritt 1) bereitgestellte Gasstrom neben Phosgen ≥ 1 Vol-% bis ≤ 20 Vol-%, bevorzugt ≥ 2 Vol-% bis ≤ 12 Vol-% und besonders bevorzugt ≥ 3 Vol-% bis ≤ 10 Vol-% CO enthält.

4. Verfahren gemäß einem der Ansprüche 1 bis 3, **dadurch gekennzeichnet, dass** die Kondensation in Schritt 2) bei einem Druck zwischen ≥ 2,0 bar(a) und ≤ 10,0 bar(a), bevorzugt zwischen ≥ 2,5 bar(a) und ≤ 6,0 bar(a) und besonders bevorzugt zwischen ≥ 3,0 bar(a) und ≤ 5,0 bar(a) erfolgt.

5. Verfahren gemäß einem der Ansprüche 1 bis 4, **dadurch gekennzeichnet, dass** die Kondensation in Schritt 2) in mindestens 2 Stufen erfolgt, wobei die letzte Stufe zwischen ≥ -60°C und ≤ 0°C, bevorzugt zwischen ≥ -35 °C und ≤ -5°C und besonders bevorzugt zwischen ≥ -20 °C und ≤ - 10°C betrieben wird.

6. Verfahren gemäß einem der Ansprüche 1 bis 5, **dadurch gekennzeichnet, dass** der Druck in der Verdampfung in Schritt 3) oberhalb von 1,5 bar(a), bevorzugt ≥ 1,8 bar(a), besonders bevorzugt oberhalb von ≥ 2,0 bar(a) und ganz besonders bevorzugt oberhalb von ≥ 2,5 bar(a) liegt.

7. Verfahren gemäß einem der Ansprüche 1 bis 6, **dadurch gekennzeichnet, dass** das die nicht kondensierbaren Restgase aus Schritt 2) in mindestens einem Nachvereiniger mit Chlor zur weiteren Umsetzung zu Phosgen gebracht werden.

8. Verfahren gemäß einem der Ansprüche 1 bis 7, **dadurch gekennzeichnet, dass** die Energieintegration mittels eines Wärmeträgerkreislaufs zwischen mindestens einem Kondensator aus Schritt 2) und mindestens einem Verdampfer aus Schritt 3) erfolgt.

9. Vorrichtung zur Herstellung von gereinigtem Phosgen-Dampf aus der Umsetzung von Chlor mit Kohlenmonoxid umfassend
- eine Kondensationseinrichtung zur teilweisen Kondensation eines Phosgen und Kohlenmonoxid umfassenden Gasstroms, die mindestens
• eine erste Zuleitung für den Phosgen und Kohlenmonoxid umfassenden Gasstrom und
• eine erste Ableitung für nicht kondensierte Gase aufweist, der eine zweite Einrichtung zur Druckhaltung zugeordnet ist,
- eine Verdampfungseinrichtung zur Verdampfung der in der Kondensationseinrichtung kondensierten Flüssigkeit, die mindestens
• eine zweite Ableitung für einen gasförmigen Phosgenstrom aufweist,
- eine von der Kondensationseinrichtung abgehende und in die Verdampfungseinrichtung mündende Verbindungsleitung zur Förderung der in der Kondensationseinrichtung kondensierten Flüssigkeit, wobei der Verbindungsleitung optional eine erste Einrichtung zur Druckhaltung zugeordnet ist und
- eine Einrichtung zur Übertragung von Wärme aus der Kondensationseinrichtung in die Verdampfungseinrichtung umfassend
• mindestens eine Wärmeübertragungsfläche, die durch eine der Kondensationseinrichtung und der Verdampfungseinrichtung gemeinsame Wandung gebildet wird oder
• mindestens einen Sekundärkreislauf der mit beiden Räumen jeweils mindestens eine gemeinsame Wandung aufweist und der zur Beinhaltung eines Wärmeträgermediums eingerichtet ist,
**dadurch gekennzeichnet dass** die Kondensationseinrichtung so eingerichtet ist, dass ein Überdruck gegenüber der Verdampfungseinrichtung von ≥ 0,2 und ≤ 6,0 bar erreichbar ist.

10. Vorrichtung gemäß Anspruch 9, **dadurch gekennzeichnet, dass** der zweiten Ableitung für einen gasförmigen Phosgenstrom eine dritte Einrichtung zur Druckhaltung zugeordnet ist.

11. Vorrichtung gemäß einem der Ansprüche 9 oder 10, **dadurch gekennzeichnet, dass** es sich bei der Kondensationseinrichtung und der Verdampfungseinrichtung jeweils um die Rohrseite eines oder mehrerer Rohrbündelwärmetauscher handelt.

12. Vorrichtung gemäß Anspruch 11, **dadurch gekennzeichnet, dass** die Einrichtung zur Übertragung von Wärme mehrere Leitungen und mindestens eine Zwangsförderungseinrichtung umfasst, wobei diese derart angeordnet sind, dass sich unter Einbeziehung der Mantelseiten der Rohrbündelwärmetauscher ein Kreislauf ergibt, in dem ein Wärmeträgermedium zirkuliert werden kann.

## Claims

1. Process for producing purified phosgene vapour, comprising the steps of
1) providing a gas stream comprising phosgene and carbon monoxide, obtainable from the reaction of chlorine with carbon monoxide,
2) condensing the gas stream in one or more stages and removing uncondensable residual gases,
3) evaporating the liquid phosgene obtained in step 2) in one or more stages and optionally superheating the phosgene vapour generated,
wherein there is energy integration between one or more of the condensation stages of step 2) and one or more of the evaporation stages in step 3) and the pressure in the last condensation stage is between ≥ 0.2 and ≤ 6.0 bar higher than in the first evaporation stage.

2. Process according to Claim 1, **characterized in that** the pressure in the last condensation stage is between ≥ 0.3 and ≤ 4.5 bar, preferably between ≥ 0.5 and ≤ 3.0 bar, more preferably between ≥ 0.8 and ≤ 2.5 bar and most preferably between ≥ 1.0 and ≤ 2.5 bar higher than in the first evaporation stage.

3. Process according to either of Claims 1 and 2, **characterized in that** the gas stream provided in step 1), as well as phosgene, contains ≥ 1% by volume to ≤ 20% by volume, preferably ≥ 2% by volume to ≤ 12% by volume and more preferably ≥ 3% by volume to ≤ 10% by volume of CO.

4. Process according to any of Claims 1 to 3, **characterized in that** the condensation in step 2) is effected at a pressure between ≥ 2.0 bar(a) and ≤ 10.0 bar(a), preferably between ≥ 2.5 bar(a) and ≤ 6.0 bar(a) and more preferably between ≥ 3.0 bar(a) and ≤ 5.0 bar(a).

5. Process according to any of Claims 1 to 4, **characterized in that** the condensation in step 2) is effected in at least 2 stages, where the last stage is implemented between ≥ -60°C and ≤ 0°C, preferably between ≥ -35°C and ≤ -5°C and more preferably between ≥ -20°C and ≤ -10°C.

6. Process according to any of Claims 1 to 5, **characterized in that** the pressure in the evaporation in step 3) is above 1.5 bar (a), preferably ≥ 1.8 bar(a), more preferably above ≥ 2.0 bar(a) and most preferably above ≥ 2.5 bar(a).

7. Process according to any of Claims 1 to 6, **characterized in that** the uncondensable residual gases from step 2) are subjected to further conversion to phosgene in at least one recombiner with chlorine.

8. Process according to any of Claims 1 to 7, **characterized in that** the energy integration is effected by means of a heat transfer circuit between at least one condenser from step 2) and at least one evaporator from step 3).

9. Apparatus for producing purified phosgene vapour from the reaction of chlorine with carbon monoxide, comprising
- a condensation unit for partial condensation of a gas stream comprising phosgene and carbon monoxide, having at least
• a first inlet for the gas stream comprising phosgene and carbon monoxide and
• a first outlet for uncondensed gases, to which a second pressure-retaining device has been assigned,
- an evaporation unit for evaporating the liquid condensed in the condensation unit, having at least
• a second outlet for a gaseous phosgene stream,
- a connecting conduit that departs from the condensation unit and opens into the evaporation unit for conveying the liquid condensed in the condensation unit, where a first pressure-retaining device has optionally been assigned to the connecting conduit, and
- a device for transfer of heat from the condensation unit to the evaporation unit, comprising
• at least one heat transfer surface which is formed by a wall common to the condensation unit and the evaporation unit or
• at least one secondary circuit having at least one common wall with each of the two spaces and set up to accommodate a heat transfer medium,
**characterized in that** the condensation unit is set up such that a positive pressure relative to the evaporation unit of ≥ 0.2 and ≤ 6.0 bar is attainable.

10. Apparatus according to Claim 9, **characterized in that** a third pressure-retaining device is assigned to the second outlet for a gaseous phosgene stream.

11. Apparatus according to either of Claims 9 and 10, **characterized in that** the condensation unit and the evaporation unit are each the tube side of one or more shell and tube heat exchangers.

12. Apparatus according to Claim 11, **characterized in that** the heat transfer device comprises multiple conduits and at least one forced conveying unit, these being arranged, with inclusion of the shell sides of the shell and tube heat exchangers, in such a way as to result in a circuit in which a heat transfer medium can be circulated.

## Revendications

1. Procédé de préparation de vapeur purifiée de phosgène, comprenant les étapes de :
1) mise à disposition d'un flux gazeux, comprenant du phosgène et du monoxyde de carbone, pouvant être obtenu à partir de la transformation de chlore avec du monoxyde de carbone,
2) condensation en un ou plusieurs étages du flux gazeux et séparation de gaz résiduels non condensables,
3) évaporation en un ou plusieurs étages du phosgène liquide obtenu dans l'étape 2) et le cas échéant surchauffe de la vapeur de phosgène obtenue,
une intégration d'énergie entre un ou plusieurs des étages de condensation de l'étape 2) et un ou plusieurs des étages de condensation dans l'étape 3) ayant lieu et la pression dans le dernier étage de condensation étant supérieure d'entre ≥ 0,2 et ≤ 6,0 bars à celle dans le premier étage d'évaporation.

2. Procédé selon la revendication 1, **caractérisé en ce que** la pression dans le dernier étage de condensation est supérieure d'entre ≥ 0,3 et ≤ 4,5 bars, de préférence d'entre ≥ 0,5 et ≤ 3,0 bars, de manière particulièrement préférée d'entre ≥ 0,8 et ≤ 2,5 bars et de manière tout particulièrement préférée d'entre ≥ 1,0 et ≤ 2,5 bars à celle dans le premier étage d'évaporation.

3. Procédé selon l'une quelconque des revendications 1 ou 2, **caractérisé en ce que** le flux gazeux mis à disposition dans l'étape 1) contient, outre du phosgène, ≥ 1% en volume à ≤ 20% en volume, de préférence ≥ 2% en volume à ≤ 12% en volume et de manière particulièrement préférée ≥ 3% en volume à ≤ 10% en volume de CO.

4. Procédé selon l'une quelconque des revendications 1 à 3, **caractérisé en ce que** la condensation dans l'étape 2) a lieu à une pression entre ≥ 2,0 bars (a) et ≤ 10,0 bars(a), de préférence entre ≥ 2,5 bars (a) et ≤ 6,0 bars (a) et de manière particulièrement préférée entre ≥ 3,0 bars (a) et ≤ 5,0 bars(a).

5. Procédé selon l'une quelconque des revendications 1 à 4, **caractérisé en ce que** la condensation dans l'étape 2) a lieu dans au moins 2 étages, le dernier étage fonctionnant entre ≥ -60°C et ≤ 0°C, de préférence entre ≥ -35°C et ≤ -5°C et de manière particulièrement préférée entre ≥ -20°C et ≤ -10°C.

6. Procédé selon l'une quelconque des revendications 1 à 5, **caractérisé en ce que** la pression dans l'évaporation dans l'étape 3) est supérieure à 1,5 bar (a), de préférence ≥ 1,8 bar(a), de manière particulièrement préférée supérieure à ≥ 2,0 bars(a) et de manière tout particulièrement préférée supérieure à ≥ 2,5 bars(a).

7. Procédé selon l'une quelconque des revendications 1 à 6, **caractérisé en ce que** les gaz résiduels non condensables de l'étape 2) sont amenés à une nouvelle transformation en phosgène dans au moins un post-dispositif de recombinaison avec du chlore.

8. Procédé selon l'une quelconque des revendications 1 à 7, **caractérisé en ce que** l'intégration d'énergie a lieu au moyen d'un circuit caloporteur entre au moins un condensateur de l'étape 2) et au moins un évaporateur de l'étape 3).

9. Dispositif pour la préparation de vapeur purifiée de phosgène provenant de la transformation de chlore avec le monoxyde de carbone, comprenant
- un dispositif de condensation destiné à la condensation partielle d'un flux gazeux comprenant du phosgène et du monoxyde de carbone qui présente au moins
- une première conduite d'alimentation pour le flux gazeux comprenant du phosgène et du monoxyde de carbone et
- une première conduite d'évacuation pour des gaz non condensés, à laquelle est associé un deuxième dispositif pour le maintien de la pression,
- un dispositif d'évaporation destiné à l'évaporation du liquide condensé dans le dispositif de condensation qui présente au moins
- une deuxième conduite d'évacuation pour un flux gazeux de phosgène,
- une conduite de liaison partant du dispositif de condensation et débouchant dans le dispositif d'évaporation, destinée à transporter le liquide qui condense dans le dispositif de condensation, un premier dispositif destiné au maintien de la pression étant éventuellement associé à la conduite de liaison et
- un dispositif destiné au transfert de chaleur provenant du dispositif de condensation dans le dispositif d'évaporation comprenant
- au moins une surface de transfert de chaleur, qui est formée par une paroi commune du dispositif de condensation et du dispositif d'évaporation ou
- au moins un circuit secondaire qui présente à chaque fois au moins une paroi commune avec les deux espaces et qui est conçu pour comporter un milieu caloporteur,
**caractérisé en ce que** le dispositif de condensation est conçu de manière telle qu'une surpression par rapport au dispositif d'évaporation ≥ 0,2 et ≤ 6,0 bars peut être atteinte.

10. Dispositif selon la revendication 9, **caractérisé en ce qu'**un troisième dispositif pour le maintien de la pression est associé à la deuxième conduite d'évacuation pour un flux gazeux de phosgène.

11. Dispositif selon l'une quelconque des revendications 9 ou 10, **caractérisé en ce qu'**il s'agit, pour le dispositif de condensation et le dispositif d'évaporation, à chaque fois du côté tubulaire d'un ou de plusieurs échangeurs de chaleur à faisceau tubulaire.

12. Dispositif selon la revendication 11, **caractérisé en ce que** le dispositif pour le transfert de chaleur comprend plusieurs conduites et au moins une conduite d'alimentation forcée, celles-ci étant agencées de manière telle qu'on obtient, compte tenu des côtés enveloppe de l'échangeur de chaleur à faisceau tubulaire, un circuit dans lequel un milieu caloporteur peut être mis en circulation.
